# FASCICULE DE BREVET EUROPEEN

(11) **EP 0 806 403 B1**
(45) Date de publication et mention de la délivrance du brevet: **26.07.2000**
(21) Numéro de dépôt: 97400886.4
(22) Date de dépôt: 21.04.1997
(51) Int. Cl.: C04B 38/00, G01N 33/24, B32B 7/02

(54) **Procédé de fabrication d'un matériau poreux inhomogène**
Verfahren zur Herstellung eines inhomogenen porösen Materials
Process for the preparation of an inhomogeneous porous material

(30) Priorité: 09.05.1996 FR 9605888
(43) Date de publication de la demande: 12.11.1997
(73) Titulaire: INSTITUT FRANCAIS DU PETROLE, 92852 Rueil-Malmaison Cedex (FR)
(72) Inventeur: Deruyter, Christian, 92500 Rueil-Malmaison (FR); Moulu, Jean-Claude, 78410 Aubergenville (FR); Kalaydjian, François, 92500 Rueil-Malmaison (FR)

(56) Documents cités:
- EP-A- 0 228 631
- CH-A- 603 211
- US-A- 5 297 420
- DATABASE WPI Section Ch, Week 8505 Derwent Publications Ltd., London, GB; Class L01, AN 85-028320 XP002024598 & JP 59 223 278 A (INA SEITO KK) , 15 Décembre 1984
- REVUE DE L'INSTITUT FRANCAIS DU PETROLE, vol. 47, no. 5, 1 Septembre 1992, pages 685-701, XP000315205 FASSI-FIHRI O ET AL: "ETUDE DE LA MOUILLABILITE DES ROCHES RESERVOIR A L'ECHELLE DU PORE PAR CRYOMICROSCOPIE ELECTRONIQUE A BALAYAGE"
- AICHE JOURNAL, vol. 23, no. 6, Novembre 1977, pages 786-794, XP000617508 MORANVILLE M. B. & AL.: "Dispersion in layered porous media"

## Description

La présente invention concerne un procédé de fabrication d'un matériau poreux inhomogène et un modèle physique transparent permettant notamment de simuler et de visualiser en laboratoire le comportement d'échantillons vis-à-vis de fluides injectés.

Un tel modèle est utile par exemple pour l'étude en laboratoire du déplacement de fluides polyphasiques dans un milieu poreux de caractéristiques analogues à celles des roches de gisements souterrains pouvant recéler des hydrocarbures.

On sait dans l'industrie fabriquer pour des applications très variées, des matériaux agglomérés que l'on obtient par exemple par frittage des particules de granulosité bien définie telles que des poudres de céramique, de micro-billes de verre, de métal etc. On forme ainsi des blocs dont la porosité est relativement homogène, ou bien des pièces composites qui combinent des agglomérés réalisés à partir de différentes substances. Un exemple de procédé connu de fabrication d'un matériau inhomogène pour isolant thermique par frittage de poudres métalliques et de poudres de céramique, est décrit par exemple dans le brevet WO/8505352.

Le procédé selon l'invention a pour objet la fabrication d'un milieu inhomogène dont la porosité varie par une transition rapide d'un endroit à un autre du milieu, par juxtaposition d'au moins deux matériaux poreux.

Il est caractérisé en ce que l'on fait varier progressivement la température de l'ensemble formé par ces matériaux suivant un cycle de variation prédéfini, la température maximale du cycle étant choisie inférieure à la température de fusion du matériau poreux le plus fusible mais suffisante pour obtenir une fusion partielle de ce matériau le plus fusible de manière à obtenir une interface mince présentant une variation sensiblement continue et homogène de la porosité entre les deux matériaux.

La méthode selon l'invention permet d'obtenir une transition rapide de porosité, sans formation entre les matériaux d'une barrière capillaire continue ou discontinue ou d'une interzone de passage préférentiel pour les fluides.

Le ramollissement lent obtenu entraîne une soudure plus ou moins homogène des grains les uns avec les autres sans obturation des interstices entre eux.

Suivant un premier mode de réalisation, on applique le cycle de variation de température à un milieu formé par juxtaposition d'un matériau minéral naturellement poreux tel que du grès et d'un matériau composite obtenu par agglomération de poudre.

Suivant un autre mode de réalisation, on applique le cycle de variation de température à un milieu formé par juxtaposition de deux matériaux composites.

Suivant un autre mode de réalisation, on applique le cycle de variation de température à un milieu formé par juxtaposition de deux matériaux minéraux de porosités différentes avec interposition entre eux d'un matériau composite.

Suivant un mode de réalisation, on réalise un bloc inhomogène en juxtaposant dans un récipient par exemple au moins deux volumes de matériaux de porosités et de températures de fusion différentes, et on place le récipient dans un four dont la température est programmée pour s'élever progressivement jusqu'à une température suffisante pour le ramollissement du matériau poreux de plus faible température de fusion pendant un premier intervalle de temps, s'y stabiliser durant un deuxième intervalle temps défini et décroître plus lentement jusqu'à la température ambiente, durant un troisième intervalle de temps. Le matériau poreux qui s'est ramolli constitue un moyen de collage des matériaux qui empêche la formation par exemple d'une quelconque lame d'air qui constituerait une barrière capillaire, ni formation d'une interzone constituant un passage préférentiel pour les fluides.

On peut utiliser par exemple une juxtaposition d'un matériau poreux naturel tel que du grès, et d'un matériau composite tel que du verre fritté,par exemple et dans ce cas, la température de stabilisation du four est choisie de l'ordre de 750°C et les durées respectives du premier, du deuxième et du troisième intervalle de temps sont choisis respectivement de l'ordre de 3h, 1h et 20h.

Le matériau composite peut être également un aggloméré de billes de verre de porosité choisie.

Le procédé selon l'invention permet d'obtenir simplement et à faible coût un modèle de milieu inhomogène avec une configuration choisie de zones de porosités bien définies permettant la mise au point de méthodes de récupération assistée d'hydrocarbures dans des gisements souterrains.

D'autres caractéristiques et avantages du procédé selon l'invention, apparaîtront à la lecture de la description ci-après en se référant aux dessins annexés où
- la Fig. 1 montre une courbe de variation de la température que l'on fait subir à un assemblage de matériaux pour obtenir la formation de transitions capillaires continues à chaque interface;
- la Fig.2 montre un assemblage d'un échantillon d'un premier matériau inclus dans un bloc dans un deuxième matériau; et
- la Fig.3 montre la courbe de variation de la porosité P suivant l'axe OX avec deux zones de transition rapides aux interfaces entre les deux matériaux.

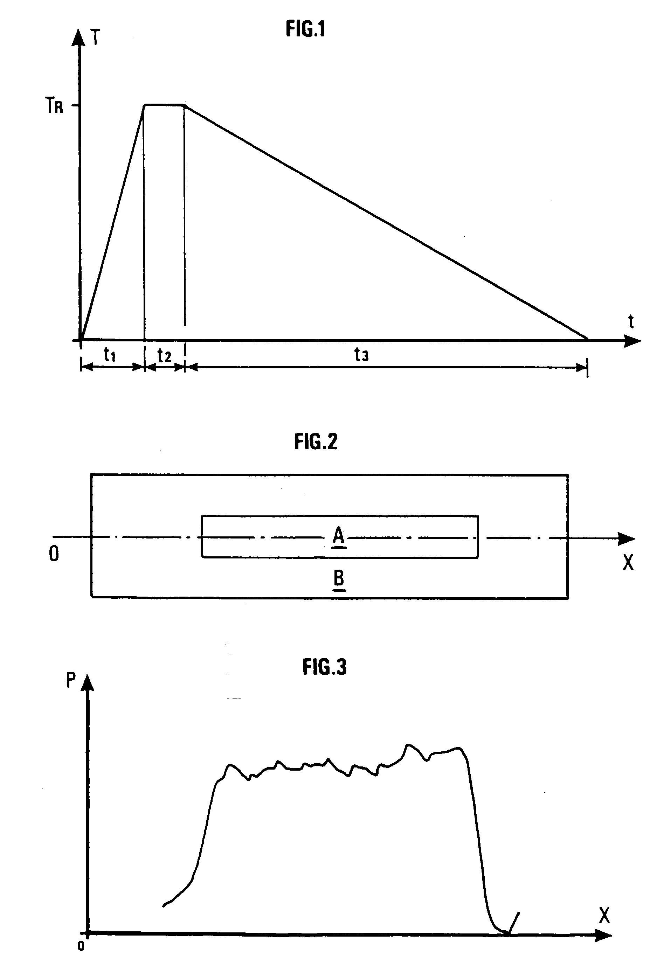

On a réalisé par exemple des blocs en juxtaposant dans un récipient un premier et un deuxième matériau. Le récipient est placé dans un four sous le contrôle d'un micro-ordinateur programmé pour faire suivre à la température du four une variation de température en fonction du temps représentée à la Fig.1. Dans un premier temps, la température du four est élevée progressivement durant un temps t1 depuis la température ambiante jusqu'à une température TR inférieure à la température de fusion des deux matériaux mais suffisante pour obtenir un ramollissement et/ou fusion partielle de l'un des matériaux Cette température est maintenue durant un temps t2 puis elle décroit pour revenir progressivement jusqu'à la température ambiante. Ce retour est plus lent, il s'étale sur un intervalle de temps t3.

A titre d'exemple, on peut inclure comme le montre la Fig.2, un volume de poudre de pyrex agglomérée A dont la température de fusion est de l'ordre de 800°C, à l'intérieur d'un barreau B de grès (grès des Vosges ou grès de Fontainebleau par exemple). La température TR est choisie de l'ordre de 750°C et les intervalles de temps t1, t2, t3 respectivement de l'ordre de 3h, 1h et 20h.

Le résultat est la création d'une zone mince de transition capillaire rapide, qui est continue tout le long de l'interface, comme le montre la courbe de variation de la porosité (Fig.3) aux interfaces entre les deux échantillons A, B schématisés sur la Fig.2.

On peut obtenir ainsi une série de strates de porosités et perméabilités différentes, propres à la réalisation d'un modèle de laboratoire pour tester le déplacement de fluides polyphasiques dans des milieux plus ou moins perméables tels qu'on en trouve dans des gisements pétrolifères. On taille et on façonne par exemple un échantillon de grès sur lequel on étale un autre matériau en poudre que l'on tasse bien avec une pression plus ou moins forte selon le degré de porosité que l'on veut obtenir. Un nouvel échantillon de roche plus ou moins poreuse peut être superposé au bloc de poudre précédent, lui-même au besoin recouvert d'un nouvel aggloméré de poudre, soit de même nature que le précédent, soit éventuellement différent On peut ainsi à volonté obtenir un milieu stratifié en multipliant les couches alternées en changeant au besoin la nature des roches et des matériaux en poudre utilisés et/ou la technique de tassement des poudres utilisées.

On choisit toujours la température TR inférieure à, mais proche de la température de fusion du matériau le plus fusible de façon à obtenir cette fusion partielle.

Comme autres matériaux utilisables pour réaliser des milieux hétérogènes de porosités et de perméabilités différentes, on peut utiliser également des matériaux composites tels que des agglomérés de particules de verre de granulosité bien définie, du béton cellulaire etc.

On réalise des milieux inhomogènes par association soit de matériaux composites de températures de ramollissement différentes, soit d'échantillons de roche plus ou moins poreux et de matériau composites, soit encore d'échantillons de roche de porosité différentes entre lesquels on interpose de préférence une mince couche d'un composite qui, par sa fusion partielle élimine toute lame d'air entre eux, susceptible de créer une barrière étanche, continue ou discontinue.

## Revendications

1. Procédé de fabrication d'un milieu inhomogène dont la porosité varie de façon discontinue d'un endroit à un autre de son volume, par association de matériaux poreux, caractérisé en ce que l'on fait varier progressivement la température de l'ensemble formé par ces matériaux associés suivant un cycle de variation prédéfini, la température maximale du cycle étant choisie inférieure à la température de fusion du matériau poreux le plus fusible mais suffisante pour obtenir une fusion partielle de ce matériau le plus fusible de manière à obtenir une interface mince présentant une variation sensiblement continue et homogène de la porosité entre les deux matériaux.

2. Procédé selon la revendication 1, caractérisé en ce que l'on applique le cycle de variation de température à un milieu formé par juxtaposition d'un matériau minéral naturellement poreux tel que du grès et d'un matériau composite.

3. Procédé selon la revendication 1, caractérisé en ce que l'on applique le cycle de variation de température à un milieu formé par juxtaposition de deux matériaux composites.

4. Procédé selon la revendication 1, caractérisé en ce que l'on applique le cycle de variation de température à un milieu formé par juxtaposition de deux matériaux minéraux de porosité différentes avec interposition entre eux d'une couche d'un matériau composite.

5. Procédé selon l'une des revendications précédentes, caractérisé en ce que l'on réalise un bloc inhomogène en juxtaposant dans un récipient au moins un volume d'un premier matériau avec au moins un volume d'un deuxième matériau présentant une température de ramollissement différente de celle du premier matériau, et on place le récipient dans un four dont la température est programmée pour s'élever progressivement jusqu'à une température suffisante pour le ramollissement du matériau composite pendant un premier intervalle de temps (t1), s'y stabiliser durant un deuxième intervalle de temps défini (t2) et décroître plus lentement jusqu'à la température ambiante, durant un troisième intervalle de temps (t3).

6. Procédé selon la revendication 5, caractérisé en ce que le premier matériau est un échantillon de roche poreuse, le deuxième matériau est constitué de verre fritté, la température de stabilisation du four est choisie de l'ordre de 750°C et les durées respectives du premier, du deuxième et du troisième intervalle de temps sont choisis respectivement de l'ordre de 3h, 1h et 20h.

7. Procédé selon l'une des revendications précédentes, caractérisé en ce que l'on utilise un matériau composite tel qu'un aggloméré de particules de verre de porosité choisie.

8. Application du procédé selon l'une des revendications précédentes, à la réalisation d'un modèle représentatif d'un milieu inhomogène avec des zones de porosités et perméabilités différentes pour la mise au point de méthodes de récupération assistée d'hydrocarbures dans des gisements souterrains.

9. Modèle représentatif d'un milieu inhomogène tel qu'une zone-réservoir souterraine, caractérisé en ce qu'il comporte la juxtaposition d'au moins un volume d'un premier matériau poreux et d'au moins un volume d'un deuxième matériau, avec entre eux une interface mince présentant une variation sensiblement continue et homogène de la porosité entre les deux matériaux, obtenue en portant l'ensemble à une température choisie inférieure à la température de fusion des deux matériaux mais suffisante pour obtenir une fusion partielle d'un des matériaux aux interfaces entre les matériaux juxtaposés.

10. Modèle selon la revendication 9, caractérisé en ce que l'un au moins des deux matériaux juxtaposés est un échantillon de roche poreuse.

11. Modèle selon la revendication 9 ou 10, caractérisé en ce que l'un au moins des deux matériaux juxtaposés est un matériau composite.

## Patentansprüche

1. Verfahren zur Herstellung eines inhomogenen Mediums bzw. einer inhomogenen Umgebung, dessen bzw. deren Porosität diskontinuierlich von einem Ort ihres Volumens zum anderen variiert, durch Zuordnung poröser Materialien, dadurch gekennzeichnet, daß man allmählich die Temperatur der durch diese zugeordneten Materialien gebildeten Anordnung entsprechend einem vordefinierten Variationszyklus variieren läßt, wobei die Maximaltemperatur des Zyklus kleiner als die Schmelztemperatur des schmelzbarsten porösen Materials, jedoch ausreichend gewählt wird, um ein partielles Schmelzen dieses schmelzbarsten Materials zu erhalten, derart, daß man eine dünne Grenzfläche erhält, die eine im wesentlichen kontinuierliche und homogene Veränderung der Porosität zwischen diesen beiden Materialien aufweist.

2. Verfahren nach Anspruch 1, dadurch gekennzeichnet, daß man den Temperaturveränderungszyklus bei einem Medium anwendet, das durch Nebenaneinanderanordnung eines natürlichen porösen keramischen Materials, wie Sandstein, und eines Verbundmaterials gebildet ist.

3. Verfahren nach Anspruch 1, dadurch gekennzeichnet, daß man den Temperaturveränderungszyklus auf ein Medium anwendet, das durch Nebeneinanderanordnung zweier Verbundmaterialien gebildet ist.

4. Verfahren nach Anspruch 1, dadurch gekennzeichnet, daß man den Temperaturveränderungszyklus auf ein Medium anwendet, das durch die Nebeneinanderanordnung von zwei mineralischen Materialien unterschiedlicher Porosität unter Zwischenschaltung einer Schicht eines Verbundmaterials zwischen diesen gebildet ist.

5. Verfahren nach einem der vorhergehenden Ansprüche, dadurch gekennzeichnet, daß man einen inhomogenen Block realisiert, indem man in einem Behälter wenigstens ein Volumen eines ersten Materials mit wenigstens einem Volumen eines zweiten Materials, das eine Erweichungstemperatur unterschiedlich zu der des ersten Materials aufweist, nebeneinander anordnet und man den Behälter in einen Ofen gibt, dessen Temperatur programmiert ist, um allmählich bis auf eine ausreichende Temperatur für die Erweichung des Verbundmaterials während eines ersten Zeitintervalls (t1) zu steigen, sich dort während eines zweiten definierten Zeitintervalls (t2) zu stabilisieren und langsamer bis auf die Umgebungstemperatur während eines dritten Zeitintervalls (t3) abzunehmen.

6. Verfahren nach Anspruch 5, dadurch gekennzeichnet, daß das erste Material eine Probe porösen Gesteins ist, das zweite Material aus Sinterglas gebildet ist, wobei die Stabilisierungstemperatur des Ofens in der Größenordnung von 750°C gewählt ist und die jeweiligen Dauern des ersten, zweiten und dritten Zeitintervalls jeweils in der Größenordnung von 3 Stunden, 1 Stunde und 20 Stunden gewählt sind.

7. Verfahren nach einem der vorhergehenden Ansprüche, dadurch gekennzeichnet, daß man ein Verbundmaterial, beispielsweise ein Agglomerat aus Glaspartikeln gewählter Porosität, verwendet.

8. Anwendung des Verfahrens nach einem der vorhergehenden Ansprüche auf die Realisierung eines Modells, das repräsentativ für eine inhomogene Umgebung mit Porositätszonen und unterschiedlichen Permeabilitäten für die Verwirklichung von unterstützten Rückgewinnungsverfahren für Kohlenwasserstoffe in unterirdischen Lagerstätten ist.

9. Für ein inhomogenes Medium, beispielsweise eine unterirdische Speicherzone repräsentatives Modell, gekennzeichnet durch die Nebeneinanderanordnung wenigstens eines Volumens eines ersten porösen Materials und wenigstens eines Volumens eines zweiten Materials unter Zwischenschaltung einer dünnen Grenzfläche zwischen diesen, welche eine im wesentlichen kontinuierliche und homogene Veränderung der Porosität zwischen den beiden Materialien aufweist, die erhalten wird, indem man die Gesamtanordnung auf eine gewählte Temperatur unterhalb der Schmelztemperatur der beiden Materialien bringt, die jedoch ausreichend ist, um ein partielles Schmelzen eines der Materialien an den Grenzflächen zwischen den nebeneinander angeordneten Materialien zu erhalten.

10. Modell nach Anspruch 9, dadurch gekennzeichnet, daß wenigstens eines der beiden nebeneinander angeordneten Materialien eine poröse Gesteinsprobe ist.

11. Verfahren nach Anspruch 9 oder 10, dadurch gekennzeichnet, daß wenigstens eines der beiden nebeneinander angeordneten Materialien ein Verbundmaterial ist.

## Claims

1. Process for manufacturing an inhomogeneous medium the porosity of which varies discontinuously from one place to another in its volume, through association of porous materials, characterised in that the temperature of the assembly formed by these associated materials is made to vary progressively according to a predefined variation cycle, the maximum temperature of the cycle being selected less than the melting temperature of the most fusible porous material but sufficient to obtain partial melting of this most fusible material so as to obtain a thin interface exhibiting a substantially continuous and homogeneous variation in the porosity between the two materials.

2. Process according to claim 1, characterised in that the temperature variation cycle is applied to a medium formed by juxtaposition of a naturally porous mineral material such as sandstone and a composite material.

3. Process according to claim 1, characterised in that the temperature variation cycle is applied to a medium formed by juxtaposition of two composite materials.

4. Process according to claim 1, characterised in that the temperature variation cycle is applied to a medium formed by juxtaposition of two mineral materials of different porosity with a layer of a composite material interposed between them.

5. Process according to one of the preceding claims, characterised in that an inhomogeneous block is produced by juxtaposing in a container at least one volume of a first material with at least one volume of a second material having a different softening temperature to that of the first material, and the container is placed in a furnace the temperature of which is programmed to rise progressively up to a sufficient temperature for softening of the composite material during a first interval of time (t1), to stabilise there during a defined second interval of time (t2) and to decrease more slowly down to the ambient temperature during a third interval of time (t3).

6. Process according to claim 5, characterised in that the first material is a sample of porous rock, the second material is constituted by sintered glass, the furnace stabilisation temperature is selected of the order of 750°C and the respective lengths of the first, second and third intervals of time are selected respectively of the order of 3h, 1h and 20h.

7. Process according to one of the preceding claims, characterised in that a composite material is used such as an agglomerate of particles of glass of selected porosity.

8. Application of the process according to one of the preceding claims to the production of a representative model of an inhomogeneous medium with zones of different porosities and permeabilities for the development of methods for assisted recovery of hydrocarbons from subterranean deposits.

9. Representative model of an inhomogeneous medium such as a subterranean reservoir zone, characterised in that it comprises the juxtaposition of at least one volume of a first porous material and at least one volume of a second material with between them a thin interface exhibiting a substantially continuous and homogeneous variation in porosity between the two materials, obtained by bringing the assembly to a selected temperature of less than the melting temperature of the two materials but sufficient to obtain partial melting of one of the materials at the interfaces between the juxtaposed materials.

10. Model according to claim 9, characterised in that one at least of the two juxtaposed materials is a sample of porous rock.

11. Model according to claim 9 or 10, characterised in that one at least of the two juxtaposed materials is a composite material.
